# EUROPÄISCHE PATENTSCHRIFT

(11) **EP 3 773 889 B1**
(45) Veröffentlichungstag und Bekanntmachung des Hinweises auf die Patenterteilung: **06.04.2022**
(21) Anmeldenummer: 20706830.5
(22) Anmeldetag: 05.02.2020
(51) Int. Cl.: A61N 1/39, A61N 1/36, A61N 1/362

(54) **STIMULATIONSVORRICHTUNG FÜR DIE ELEKTROTHERAPIE SOWIE VERFAHREN ZUR ÜBERPRÜFUNG DER POLARITÄT VON KONTAKTELEKTRODEN**
STIMULATION DEVICE FOR ELECTROTHERAPY AND METHOD FOR CHECKING THE POLARITY OF CONTACT ELECTRODES
DISPOSITIF DE STIMULATION DESTINÉ À L'ÉLECTROTHÉRAPIE ET PROCÉDÉ DE SURVEILLANCE DE LA POLARITÉ D'ÉLECTRODES DE CONTACT

(30) Priorität: 12.03.2019 DE 102019106224
(43) Veröffentlichungstag der Anmeldung: 17.02.2021
(73) Patentinhaber: GS Elektromedizinische Geräte G. Stemple GmbH, 86916 Kaufering (DE)
(72) Erfinder: ULBRICH, Mark, 86916 Kaufering (DE); PEUKERT, Michael, 86916 Kaufering (DE)
(74) Vertreter: Hennicke, Ernst Rüdiger
(86) Internationale Anmeldenummer: PCT/IB2020/050928
(87) Internationale Veröffentlichungsnummer: WO 2020/183253

(56) Entgegenhaltungen:
- US-A1- 2007 100 381
- US-A1- 2014 236 248
- US-A1- 2015 273 226
- US-B2- 8 738 130

## Beschreibung

Die Erfindung betrifft eine Stimulationsvorrichtung für die Elektrotherapie, insbesondere eine Defibrillator- und/oder eine externe Herzschrittmachervorrichtung, mit mindestens zwei an geeigneten Stimulationspositionen am Körper eines Patienten applizierbaren Kontaktelektroden, über die der Körper des Patienten mit Stromimpulsen beaufschlagbar ist, wobei die erste der mindestens zwei Kontaktelektroden als Zuleitelektrode mit positiver Polarität und die zweite der mindestens zwei Kontaktelektroden als Ableitelektrode mit negativer Polarität in Bezug auf einen abgegebenen Stromimpuls wirkt, und mit einem mit den Kontaktelektroden über Leitungsverbindungen verbundenen oder verbindbaren Stromimpulserzeuger. Die Erfindung betrifft ferner ein Verfahren zur Feststellung der Polarität von am Körper eines Patienten applizierten Kontaktelektroden.

Für den Einsatz zur Defibrillation oder als externe Herzschrittmacher geeignete Stimulationsvorrichtungen werden am Körper des zu behandelnden Patienten im allgemeinen mittels zwei Kontaktelektroden angeschlossen, die an bestimmten Positionen am Oberkörper des Patienten festgelegt, beispielsweise angeklebt werden. Dabei sind die Kontaktelektroden grundsätzlich identisch, d.h. beide Elektroden können sowohl als Zuleitelektrode als auch als Ableitelektrode wirken. In Bezug auf den abgegebenen Stromimpuls wirkt dabei diejenige Elektrode mit positiver Polarität als Zuleitelektrode und die Elektrode mit negativer Polarität als Ableitelektrode.

Standard-Elektrodenpositionen für externe Schrittmacher (Pacer) und Defibrillatoren sind dabei die Positionen "anterior-posterior" und "anterior-lateral". Bei der Anordnung "anterior-lateral" soll die Polarität der anterior, d.h. rechtsseitig am vorderen, oberen Brustkorb applizierten Elektrode positiv sein, während die Polarität der lateral, also (links) seitlich unterhalb der Achselhöhle angelegten Elektrode negativ ist. Bei der Anordnung "anterior-posterior" soll die posterior, also am Rücken des Patienten angelegte Elektrode die positive Polarität haben, während die vorderseitig, also am Brustkorb angebrachte Elektrode negativ gepolt ist. Die Polarität der Elektroden ist maßgeblich für eine sinnvolle Therapie, eine Vertauschung der Polarität hebt nämlich die Koppelschwelle erheblich an, was den Therapieerfolg infrage stellen kann. Es ist nachvollziehbar, dass es gerade in Notfallsituationen, in denen die Retter unter großem zeitlichen und auch psychischen Druck stehen können, leicht unbeabsichtigt dazu kommen kann, dass die Polarität an den Elektroden zur Durchführung einer Defibrillation oder zur Herstellung einer Verbindung mit einem externen Schrittmacher vertauscht wird, womit der Behandlungserfolg verschlechtert oder ganz in Frage gestellt wird.

Aufgabe der Erfindung ist es, eine Stimulationsvorrichtung der eingangs genannten Art zu schaffen, mit der die korrekte Positionierung der Kontaktelektroden am Körper des Patienten vereinfacht wird.

Diese Aufgabe wird mit der Erfindung durch eine mit den Kontaktelektroden verbundene oder verbindbare Signal-Auswerteeinheit zur Feststellung der Applikationspositionen der Kontaktelektroden am Körper des Patienten gelöst. Hierdurch wird es nach dem erfindungsgemäßen Verfahren möglich, die Polarität von am Körper eines Patienten applizierten Kontaktelektroden der Stimulationsvorrichtung für die Elektrotherapie, insbesondere einer Defibrillator- und/oder Herzschrittmachervorrichtung zu überprüfen, indem mittels der am Körper des Patienten applizierten Kontaktelektroden körpereigene, für den jeweiligen Applikationsort der Kontaktelektroden spezifische Signale erfasst und zu einer Auswerteeinheit übermittelt werden, wobei die Auswerteeinheit aufgrund der an den Kontaktelektroden festgestellten Signale, z.B. deren Ausrichtung und/oder Größe, die tatsächliche Position der Kontaktelektroden am Körper des Patienten bestimmt. Diese kann dem Sanitäter oder Arzt mittels einer geeigneten Anzeige angezeigt werden, um diesen zu einer manuellen Umpolung der Kontaktelektroden zu veranlassen. Demgemäß kann die erfindungsgemäße Vorrichtung also eine Anzeigeeinrichtung zur Anzeige der Applikationsposition der Zuleitungselektrode und der Ableitelektrode am Körper des Patienten aufweisen.

Besonders vorteilhaft ist es, wenn die Auswerteeinheit die tatsächlich an der jeweiligen Kontaktelektrode anliegende Polarität des für die Elektrotherapie vorgesehenen Behandlungsstroms mit einer Soll-Polarität an der Kontaktelektrode an der jeweiligen tatsächlichen Position vergleicht und bei fehlender Übereinstimmung der tatsächlichen Polarität und der Soll-Polarität eine Anzeige ausgibt und/oder die tatsächliche Polarität auf die Soll-Polarität ändert. Auf diese Weise wird dem Sanitäter oder Arzt also nicht nur die jeweilige Polarität der am Körper des Patienten angebrachten Kontaktelektroden angegeben, sondern auch ggf. darauf hingewiesen, dass die Polarität vertauscht wurde bzw. in besonders bevorzugter, vorteilhafter Weise wird der Fehler beim Anschluss der Elektroden sogleich von der erfindungsgemäßen Vorrichtung selbst korrigiert. Somit kann sich das Rettungspersonal darauf verlassen, dass auch bei einer fehlerhaften Applikation (Vertauschung) der Elektroden dem Patienten die optimale Therapie zugeführt wird. Wertvolle Zeit kann hierdurch gewonnen werden.

Zur Durchführung des erfindungsgemäßen Verfahrens ist die Vorrichtung bevorzugt mit Umschaltmitteln zur Vertauschung der Leitungsverbindungen zwischen dem Stromimpulserzeuger und den Kontaktelektroden versehen. Die Umschaltmittel machen es überflüssig, die zwischen den Kontaktelektroden und dem Defibrillator/externen Schrittmacher angeschlossenen Kabelverbindungen umzustecken, wenn von der Vorrichtung eine fehlerhafte Polung der Kontaktelektroden festgestellt wurde. Die Umschaltmittel können einen Wechselschalter umfassen, bei dessen Betätigung die Umpolung schnell erfolgt. Dabei ist es besonders vorteilhaft, wenn der Wechselschalter von der Auswerteeinheit gesteuert betätigbar ist, so dass die Umschaltung ohne manuellen Eingriff des Bedienpersonals der Vorrichtung automatisch erfolgen kann.

Bei den von der Vorrichtung ausgewerteten Signalen kann es sich bevorzugt um EKG Signale handeln, die an den Positionen, an denen die Kontaktelektroden am Körper des Patienten platziert wurden, von den Elektroden abgegriffen und über die daran angeschlossenen Leitungsverbindungen an die Auswerteeinheit der Stimulationsvorrichtung übermittelt werden. Da sich die EKG-Signale je nach dem Ort der Platzierung der Kontaktelektroden signifikant unterscheiden, kann durch die Extraktion von Merkmalen aus der DE-EKG Ableitung eindeutig auf Ort der beiden Kontaktelektroden am Körper des Patienten rückgeschlossen werden. Andere Arten von Signalen, die ausgewertet werden können, um auf den jeweiligen Ort der Positionierung der Kontaktelektroden rückzuschließen, können z.B. Biopotentiale oder die Impedanz des Gewebes am Ort der angebrachten Kontaktelektroden sein. Das Dokument US-B-8 738 130 offenbart den nächstliegenden Stand der Technik.

Die Erfindung wird in den unabhängigen Ansprüchen definiert. Ausführungsformen, die nicht dem Gegenstand der unabhängigen Ansprüche entsprechen, sind nicht Bestandteil der Erfindung.

Weitere Vorteile und Merkmale der Erfindung ergeben sich aus der nachfolgenden Beschreibung und aus der Zeichnung, worin eine bevorzugte Ausführungsform der Erfindung anhand eines Beispiels näher erläutert ist. Es zeigt:
- Fig.1A: eine Darstellung der Standard-Elektrodenposition "anterior-lateral" für externe Schrittmacher und Defibrillatoren;
- Fig.1B: eine Darstellung der Standard-Elektrodenpositionen "anterior-posterior";
- Fig.2A: von der Elektrodenposition gemäß Fig.1A erhältliche EKG Signale bei korrekter Polarität (oben) und vertauschter Polarität (unten);
- Fig.2B: von der Elektrodenposition gemäß Fig.1B erhältliche EKG Signale bei korrekter Polarität (oben) und vertauschter Polarität (unten); und
- Fig.3: eine schematische Darstellung der erfindungsgemäßen Stimulationsvorrichtung zur Durchführung des erfindungsgemäßen Verfahrens.

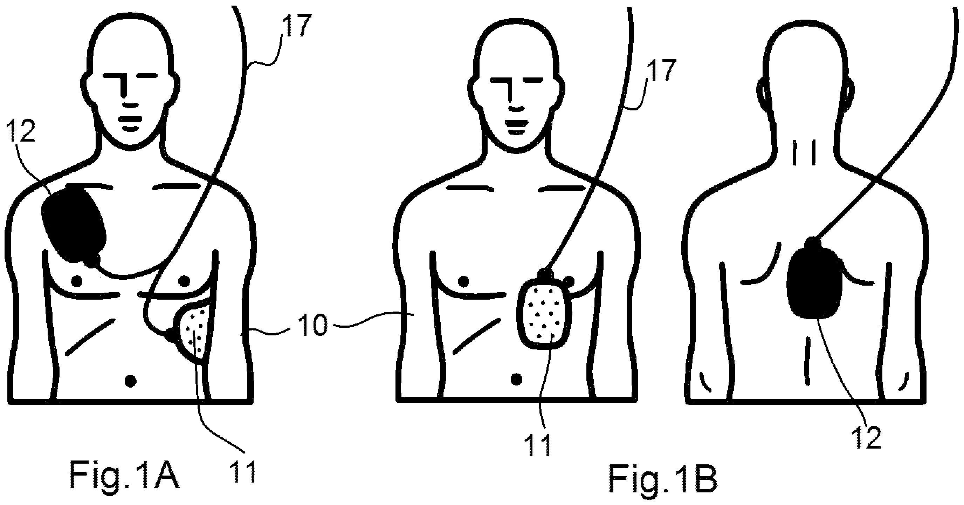

Figur 1 zeigt zwei Standardelektrodenpositionen für die Anordnung von Kontaktelektroden 11,12 am Körper eines Patienten 10, wie sie für externe Schrittmacher oder Defibrillatoren zum Einsatz kommen.

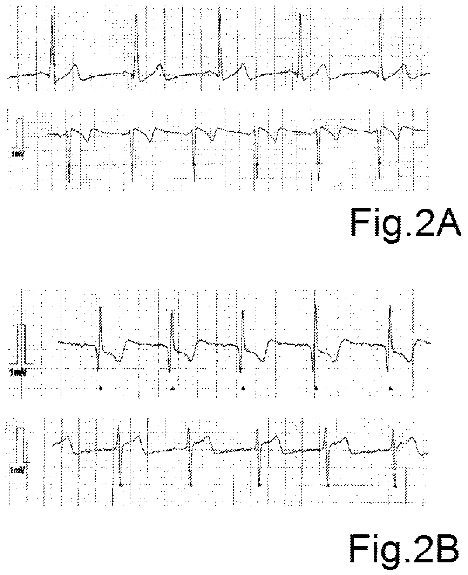

Bei Applikationen der Elektroden am Patienten kommt es nicht nur auf den korrekten Ort ihrer Anbringung, sondern auch darauf an, dass die korrekte Polarität der von dem Defibrillator oder dem Schrittmacher über die Elektroden 11,12 in den Körper des Patienten eingeleiteten elektrischen Impulse beachtet wird. Bei den beiden in den Fig.1A und 1B gezeigten Elektrodenpositionen soll jeweils die Polarität der gepunkteten Elektrode 11 negativ und die Polarität der flächig schwarz dargestellten Elektrode 12 positiv sein. Wenn man die von den Kontaktelektroden 11,12 an den gezeigten Stellen aufgezeichneten EKG-Signale ableitet und aufzeichnet, erhält man die in den Figuren 2A und 2B jeweils oben dargestellten EKG Signalverläufe, wenn die korrekte Polarität wie vorstehend erläutert eingehalten ist. Bei Vertauschung der Polarität verändert sich auch der Verlauf des EKG Antwortsignals, es erscheint dann wie in den Figuren 2A und 2B jeweils unten dargestellt. Man erkennt, dass sich durch die Vertauschung der Polarität die charakteristischen Merkmale der aufgezeichneten Wellen ändern und anhand dessen leicht erkennbar ist, ob die Polarität der beiden Kontaktelektroden der gewünschten Anordnung entspricht oder vertauscht wurde.

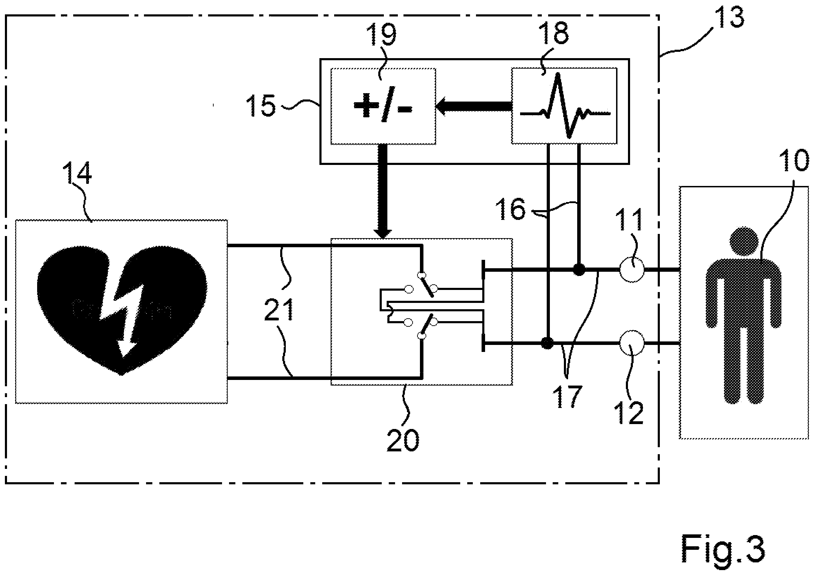

Die in Fig. 3 schematisch dargestellte, in ihrer Gesamtheit mit 13 bezeichnete Stimulationsvorrichtung umfasst neben den beiden Kontaktelektroden 11,12 einen Stromimpulserzeuger 14 und eine EKG-Auswerteeinheit 15, die über Ableitkabel 16 mit Anschlusskabeln 17 für die Kontaktelektroden 11,12 verbunden sind. Die Auswerteeinheit 15 hat eine Anzeigeeinrichtung 18 zur visuellen Darstellung der von den Kontaktelektroden 11,12 abgegriffenen EKG-Signale sowie eine Signalverarbeitungseinrichtung 19, mit der aufgrund des aufgezeichneten EKG Signals festgestellt wird, an welcher Position am Körper des Patienten sich die Zuleitelektrode 12 mit positiver Polarität und wo sich die Ableitelektrode 11 mit negativer Polarität befindet.

Wenn die Auswerteeinheit 15 eine Vertauschung der beiden Kontaktelektroden in ihren Soll-Positionen (Fig.1) am Körper des Patienten 10 feststellt, wird sie durch Betätigung eines Doppel-Wechselschalters 20 eine Vertauschung der Leitungsverbindungen 21 vom Stromimpulserzeuger 14 zu den Anschlusskabeln 17 der Kontaktelektroden 11,12 bewirken. Auf diese Weise wird also die an der jeweiligen Kontaktelektrode anliegende Polarität des für die Elektrotherapie vorgesehenen, von dem Stromimpulserzeuger 14 bereitgestellten Behandlungsstroms mit einer Soll-Polarität an der Kontaktelektrode an der jeweils tatsächlichen Position verglichen und bei fehlender Übereinstimmung von tatsächlicher Polarität und Soll-Polarität die tatsächliche auf die Soll-Polarität umgestellt. Die beiden Kontaktelektroden werden also bei dem bevorzugten Ausführungsbeispiel automatisch umgepolt, so dass dann die Zu- und Ableitung des Stroms jeweils an korrekter Stelle am Körper des Patienten 19 erfolgt, ohne dass es erforderlich ist, die Anschlusskabel zwischen den Elektroden und dem Stromimpulserzeuger zu trennen und nach Vertauschung der Polarität neu zu verbinden.

## Patentansprüche

1. Stimulationsvorrichtung für die Elektrotherapie, insbesondere Defibrillator- und/oder externe Herzschrittmachervorrichtung,
- mit mindestens zwei an geeigneten Stimulationspositionen am Körper eines Patienten applizierbaren Kontaktelektroden (11,12), über die der Körper des Patienten (10) mit Stromimpulsen beaufschlagbar ist, wobei die erste der mindestens zwei Kontaktelektroden (11,12) als Zuleitelektrode (12) mit positiver Polarität und die zweite der mindestens zwei Kontaktelektroden (11,12) als Ableitelektrode (11) mit negativer Polarität in Bezug auf einen abgegebenen Stromimpuls wirkt, und
- mit einem mit den Kontaktelektroden (11,12) über Leitungsverbindungen (21,17) verbundenen oder verbindbaren Stromimpulserzeuger (14),
**gekennzeichnet durch** eine mit den Kontaktelektroden (11,12) verbundene oder verbindbare Signal-Auswerteeinheit (15), wobei die Vorrichtung eingerichtet ist, mittels der am Körper des Patienten applizierten Kontaktelektroden (11,12) körpereigene, für den jeweiligen Applikationsort der Kontaktelektroden spezifische Signale zu erfassen und zu der Auswerteeinheit (15) zu übermitteln, wobei die Auswerteeinheit (15) zur Feststellung der tatsächlichen Applikationspositionen der Kontaktelektroden (11,12) am Körper des Patienten (10) aufgrund der mittels der Kontaktelektroden (11,12) festgestellten spezifischen Signale eingerichtet ist.

2. Vorrichtung nach Anspruch 1, **gekennzeichnet durch** eine Anzeigeeinrichtung (18) zur Anzeige der Applikationsposition der Zuleitelektrode (12) und der Ableitelektrode (11) am Körper des Patienten (10) .

3. Vorrichtung nach Anspruch 1 oder 2, **gekennzeichnet durch** Umschaltmittel (19,20) zur Vertauschung der Leitungsverbindungen (21,17) zwischen dem Stromimpulserzeuger (14) und den Kontaktelektroden (11,12).

4. Vorrichtung nach Anspruch 3, **dadurch gekennzeichnet, dass** die Umschaltmittel (19,20) einen Wechselschalter (20) umfassen.

5. Vorrichtung nach Anspruch 4, **dadurch gekennzeichnet, dass** der Wechselschalter (20) von der Auswerteeinheit (15) gesteuert betätigbar ist.

6. Vorrichtung nach einem der Ansprüche 1 bis 5, **dadurch gekennzeichnet, dass** die spezifischen Signale EKG-Signale sind.

7. Verfahren zur Überprüfung der Polarität von am Körper eines Patienten (10) applizierten Kontaktelektroden (11,12) einer Stimulationsvorrichtung (13) für die Elektrotherapie, insbesondere einer Defibrillator- und/oder Herzschrittmachervorrichtung, bei dem mittels der am Körper des Patienten applizierten Kontaktelektroden (11,12) körpereigene, für den jeweiligen Applikationsort der Kontaktelektroden spezifische Signale erfasst und zu einer Auswerteeinheit (15) übermittelt werden, wobei die Auswerteeinheit (15) aufgrund der an den Kontaktelektroden (11,12) festgestellten Signale die tatsächliche Position der Kontaktelektroden am Körper des Patienten bestimmt .

8. Verfahren nach Anspruch 7, **dadurch gekennzeichnet, dass** die Auswerteeinheit (15) die tatsächlich an der jeweiligen Kontaktelektrode anliegende Polarität des für die Elektrotherapie vorgesehenen Behandlungsstroms mit einer Soll-Polarität an der Kontaktelektrode an der jeweiligen tatsächlichen Position vergleicht und bei fehlender Übereinstimmung der tatsächlichen Polarität mit der Soll-Polarität eine Anzeige ausgibt und/oder die tatsächliche Polarität auf die Soll-Polarität ändert.

9. Verfahren nach Anspruch 7 oder 8, **dadurch gekennzeichnet, dass** die spezifischen Signale EKG-Signale sind.

## Claims

1. Stimulation device for electrotherapy, in particular defibrillator and/or external pacemaker device,
- comprising at least two contact electrodes (11, 12) that can be applied to suitable stimulation positions on the body of a patient and via which the body of the patient (10) can be subjected to current pulses, wherein the first of the at least two contact electrodes (11, 12) acts as a charging electrode (12) having positive polarity and the second of the at least two contact electrodes (11, 12) acts as a discharging electrode (11) having negative polarity with respect to an emitted current pulse, and
- comprising a current pulse generator (14) that is or can be connected to the contact electrodes (11, 12) via line connections (21, 17),
**characterised by** a signal evaluation unit (15) that is or can be connected to the contact electrodes (11, 12), wherein the device is adapted, by means of the contact electrodes (11, 12) applied to the body of the patient, to record the body's own signals specific to the respective application location of the contact electrodes and to transmit them to the evaluation unit (15), wherein the evaluation unit (15) is adapted to determine the actual application positions of the contact electrodes (11, 12) on the body of the patient (10) on the basis of the specific signals determined by means of the contact electrodes (11, 12).

2. Device according to claim 1, **characterised by** a display unit (18) for displaying the application position of the charging electrode (12) and the discharging electrode (11) on the body of the patient (10).

3. Device according to claim 1 or 2, **characterised by** switching means (19, 20) for permutation of the line connections (21, 17) between the current pulse generator (14) and the contact electrodes (11, 12).

4. Device according to claim 3, **characterised in that** the switching means (19, 20) includes a toggle switch (20).

5. Device according to claim 4, **characterised in that** the toggle switch (20) can be actuated under the control of the evaluation unit (15).

6. Device according to any one of claims 1 to 5, **characterised in that** the specific signals are ECG signals.

7. Method for checking the polarity of contact electrodes (11, 12) of a stimulation device (13) for electrotherapy, in particular a defibrillator device and/or pacemaker device, which electrodes are applied to the body of a patient (10), in which method the body's own signals specific to the respective application location of the contact electrodes are recorded by means of the contact electrodes (11, 12) applied to the body of the patient and transmitted to an evaluation unit (15), wherein the evaluation unit (15) determines the actual position of the contact electrodes (11, 12) on the body of the patient on the basis of the signals determined at the contact electrodes.

8. Method according to claim 7, **characterised in that** the evaluation unit (15) compares the polarity of the treatment current provided for electrotherapy that is actually applied at the respective contact electrode with a target polarity at the contact electrode at the respective actual position and, if the actual polarity and the target polarity do not match, outputs a display and/or changes the actual polarity to the target polarity.

9. Method according to claim 7 or 8, **characterised in that** the specific signals are ECG signals.

## Revendications

1. Dispositif de stimulation pour l'électrothérapie, en particulier dispositif défibrillateur et/ou stimulateur cardiaque externe,
- avec au moins deux électrodes de contact (11, 12) pouvant être appliquées sur le corps d'un patient à des positions de stimulation adaptées, par l'intermédiaire desquelles le corps du patient (10) peut être soumis à des impulsions électriques, dans lequel la première des au moins deux électrodes de contact (11, 12) agit en tant qu'électrode d'alimentation (12) avec une polarité positive et la seconde des au moins deux électrodes de contact (11, 12) agit en tant qu'électrode de dérivation (11) avec une polarité négative par rapport à une impulsion électrique émise, et
- avec un générateur d'impulsions électriques (14) connecté ou pouvant être connecté aux électrodes de contact (11, 12) par l'intermédiaire de connexions par câble (21, 17),
**caractérisé par** une unité d'évaluation de signaux (15) connectée ou pouvant être connectée aux électrodes de contact (11, 12), dans lequel le dispositif est conçu pour détecter, au moyen des électrodes de contact (11, 12) appliquées sur le corps du patient, des signaux propres au corps et spécifiques à l'endroit d'application respectif des électrodes de contact et pour les transmettre à l'unité d'évaluation (15), dans lequel l'unité d'évaluation (15) est configurée pour déterminer les positions d'application réelles des électrodes de contact (11, 12) sur le corps du patient (10) sur la base des signaux spécifiques déterminés au moyen des électrodes de contact (11, 12).

2. Dispositif selon la revendication 1, **caractérisé par** un dispositif d'affichage (18) pour afficher la position d'application de l'électrode d'alimentation (12) et de l'électrode de dérivation (11) sur le corps du patient (10).

3. Dispositif selon la revendication 1 ou 2, **caractérisé par** des moyens de commutation (19, 20) pour inverser les connexions par câble (21, 17) entre le générateur d'impulsions électriques (14) et les électrodes de contact (11, 12).

4. Dispositif selon la revendication 3, **caractérisé en ce que** les moyens de commutation (19, 20) comprennent un commutateur inverseur (20).

5. Dispositif selon la revendication 4, **caractérisé en ce que** le commutateur inverseur (20) peut être actionné de manière commandée par l'unité d'évaluation (15).

6. Dispositif selon l'une quelconque des revendications 1 à 5, **caractérisé en ce que** les signaux spécifiques sont des signaux ECG.

7. Procédé de contrôle de la polarité d'électrodes de contact (11, 12) appliquées sur le corps d'un patient (10) d'un dispositif de stimulation (13) pour l'électrothérapie, en particulier d'un dispositif de défibrillateur et/ou de stimulateur cardiaque, dans lequel des signaux propres au corps et spécifiques à l'endroit d'application respectif des électrodes de contact sont détectés et transmis à une unité d'évaluation (15) au moyen des électrodes de contact (11, 12), dans lequel l'unité d'évaluation (15) détermine la position réelle des électrodes de contact sur le corps du patient sur la base des signaux déterminés sur les électrodes de contact (11, 12).

8. Procédé selon la revendication 7, **caractérisé en ce que** l'unité d'évaluation (15) compare la polarité réellement appliquée à l'électrode de contact respective du courant de traitement prévu pour l'électrothérapie avec une polarité de consigne sur l'électrode de contact à la position réelle respective et, en cas de non-concordance de la polarité réelle avec la polarité de consigne, émet un affichage et/ou modifie la polarité réelle pour la faire correspondre à la polarité de consigne.

9. Procédé selon la revendication 7 ou 8, **caractérisé en ce que** les signaux spécifiques sont des signaux ECG.
